# EUROPEAN PATENT SPECIFICATION

(11) **EP 1 169 463 B1**
(45) Date of publication and mention of the grant of the patent: **15.08.2007**
(21) Application number: 00936517.2
(22) Date of filing: 26.01.2000
(51) Int. Cl.: C12N 15/82, C12N 15/40, C12N 5/10, A01H 5/00

(54) **METHOD FOR CONVEYING BNYVV RESISTANCE TO SUGAR BEET PLANTS**
VERFAHREN ZUR VERMITTLUNG VON BNYVV-RESISTENZ IN ZUCKERRÜBENPFLANZEN
METHODE POUR TRANSMETTRE LA RESISTANCE AU BNYVV AUX BETTRAVES A SUCRE

(30) Priority: 27.01.1999 EP 99200236
(43) Date of publication of application: 09.01.2002
(73) Proprietor: Ses Europe N.V./S.A., 3300 Tienen (BE)
(72) Inventor: RICHARDS, Kenneth, 67370 Pfulgriesheim (FR); JONARD, Gérard, 67000 Strasbourg (FR); GUILLEY, Hubert, 67370 Berstett (FR); VAN DUN, Cornelis, Maria, Petrus, 4708 CC Roosendaal (NL)
(74) Representative: Van Malderen, Joëlle
(86) International application number: PCT/EP2000/000609
(87) International publication number: WO 2000/044915

(56) References cited:
- WO-A-91/13159
- WO-A-98/07875
- BOUZOUBAA S ET AL: "NUCLEOTIDE SEQUENCE OF BEET NECROTIC YELLOW VEIN VIRUS RNA-1" JOURNAL OF GENERAL VIROLOGY, vol. 68, 1 January 1987 (1987-01-01), pages 615-626, XP000650387 ISSN: 0022-1317 cited in the application
- MANNERLOF, M., ET AL.: "reduced titer of BNYVV in transgenic sugar beets expressing the BNYVV coat protein" EUPHYTICA, vol. 90, no. 3, 1996, pages 293-299, XP002112359 cited in the application
- SAITO,M., ET AL.: "complete nucleotide sequence of the Japanese isolate S of beet necrotic yellow vein virus RNA and comparison with European isolates" ARCHIVES OF VIROLOGY, vol. 141, 1996, pages 2163-2175, XP002112360
- HEHN, A., ET AL.: "evidence for in vitro and in vivo autocatalytic processing of the primary translation product of beet necrotic yellow vein virus RNA 1 by a papain-like proteinase" ARCHIVES OF VIROLOGY, vol. 142, 1997, pages 1051-1058, XP002112363

## Description

The present invention relates to a method for conveying viral resistance to beet necrotic yellow vein virus (BNYVV) to a sugar beet plant. Furthermore, the invention relates to virus-resistant plants obtained according to this method, as well as to seeds and progeny derived therefrom.

Plant viruses are a serious problem for many of the major agricultural crops. Thus, BNYVV which is transmitted by the soilborne plasmodiophoromycete fungus Polymyxa betae, is the cause of the economically important disease of sugar beet (Beta vulgaris), known as rhizomania. This disease was first reported in Italy in the 1950s and has since become a major threat to sugar beet crops in most growing areas in the world.

Under field conditions BNYVV is generally confined to the subterranean portions of the plant. Infected sugar beet roots exhibit an increased proliferation and necrosis of the lateral roots, a decrease in the total mass of the main root and a consequent reduction in sugar yield. Plant death may also follow infection, particularly if the crop is infected early in season. Occasionally, the virus will ascend into the leaves where it can provide invasive vein-associated chlorotic and necrotic lesions.

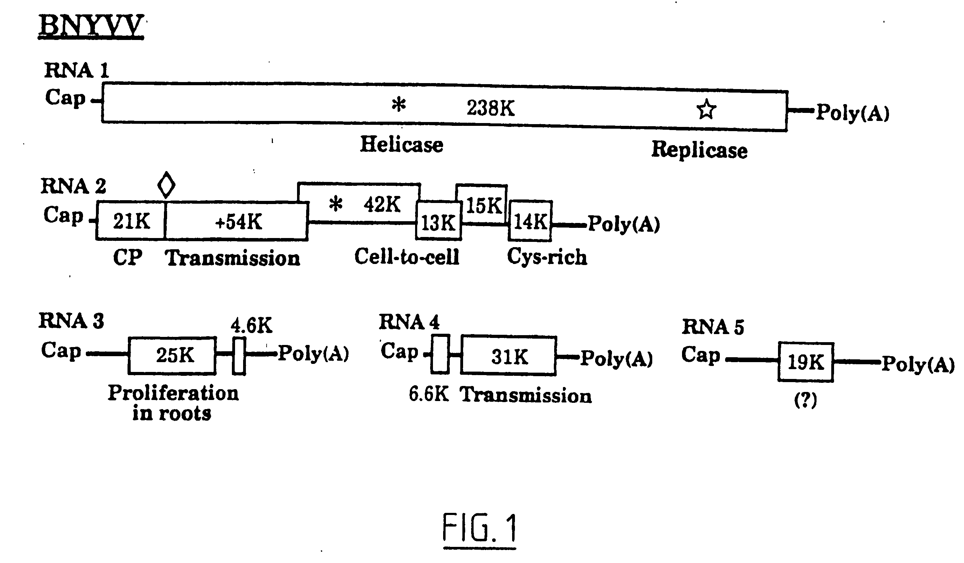

BNYVV is a type member of the benyvirus family, which are fungus-transmitted, rod-shaped viruses possessing a divided genome composed of two or more components of single-stranded (ss) RNA. Four different plus sense ssRNA species of BNYVV have been identified, which are referred to as RNAs 1 to 4 in decreasing order of size (RNA 1: 6.8 kb; RNA 2: 4.7 kb; RNA 3: 1.8 kb; RNA 4: 1.5 kb). Some Japanese isolates also contain a fifth RNA component (RNA 5: 1.45 kb). All RNAs have been cloned and sequenced. The genetic map of the viral RNAs is shown in figure 1.

All five RNAs terminate in 3' poly-A tails, and RNAs 1-4 have cap structures at their 5' termini. RNAs 1 and 2 encode basic host-independent "housekeeping" functions, while the smaller RNAs intervene specifically in the natural infection process, including vector-mediated infection of sugar beet roots, proliferation within the root system and production of rhizomania symptoms. Thus, RNA 1 has been shown to code for viral RNA polymerase activity and RNA 2 encodes the 21-kd viral coat-protein. The 3'-proximal half of RNA 2 carries a group of three successive slightly overlapping viral genes, known as the triple gene block (TGB3), that are closely similar to a cluster of three genes in other rod-shaped plant viruses, and are involved in cell to cell movement of the virus. RNA 3 is associated with massive proliferation of fine rootlets in sugar beet and facilitates the spread of the virus in root tissue, whereas RNA 4 increases the efficiency of transmission of the virus by the fungus.

Fungus-transmitted viruses, such as BNYVV may be retained in resting spores in soil for years once a field becomes infested. As there exist no effective chemical or physical methods for eliminating the virus, neither in the plants nor in the soil, the only option for the sugar beet farmer is the use of genetically resistant cultivars. Several companies have provided a number of tolerant, even partially resistant varieties, by transferring wild type tolerance genes into commercially varieties through breeding. This is, however, a very tedious and time-consuming process, generally taking a long time before useful resistant plants are obtained. In addition, under high disease pressure tolerant or partially resistant plants will develop disease symptoms because the resistance level is too low. Another problem with tolerant or only partially resistant plants lies in the fact that virus populations continue to grow resulting in the possible emergence of a BNYVV strain which may break the resistance/tolerance genes.

The rapid revolution in the areas of plant engineering has led to the development of new strategies to confer genetic resistance to viruses. Resistance to viral diseases through the introduction of portions of viral genome sequences has become a new source of resistance. The viral sequence (construct) is transformed into a plant by combining the use of appropriate cell or tissue culture techniques and a DNA delivery system such as the well known Agrobacterium tumefaciens transformation system or the direct DNA transfer mediated by chemicals like polyethylene glycol (PEG).

Known methods of inducing pathogen defense mechanisms in plants are for example described in EP 9687106.0, which relates to a method for inducing resistance to a virus comprising a TGB3 sequence. The publication describes that it was possible to induce BNYVV resistance by a method, which consists of transforming plant cells with a DNA construct corresponding to a fragment of the nucleic acid sequence of the genomic or subgenomic RNA 2 of the BNYVV. The disadvantage of this method is however, that only tolerance is obtained, and not resistance. In tolerant host plants there can still be normal levels of plant virus replication, but the plant shows little or no visible signs of infection, whereas in resistant hosts the virus replication is low or even absent.

In WO 93/25068 viral resistance in plants is induced by plant transformation with a replicase portion (RNA 1) of a plant virus genome. This publication does not refer to sugar beet, nor to BNYVV.

Sugar beet are known to be recalcitrant species in genetic engineering, complicating successful induction of BNYVV resistance. However, because of the extent and impact of the viral disease of BNYVV there is a major interest to improve the sources of genetic resistance in sugar beet plants.

The object of the present invention is, therefore, to provide means to obtain sugar beet plants, that exhibit resistance to BNYVV. Preferably, they exhibit total resistance or immunity, by combining different approaches and by using methods which do not allow the virus to replicate.

This is achieved by the invention by providing a method for conveying resistance to beet necrotic yellow vein virus (BNYVV) to a sugar beet plant, comprising the following steps:
(a) preparing a DNA fragment of at least 15 nucleotides in a sequence that is essentially homologous to the corresponding nucleotide sequence of the genomic RNA 1 of the beet necrotic yellow vein virus (BNYVV),
(b) introducing said DNA fragment, operately linked to a promoter that is active in sugar beet plants, into a sugar beet plant cell to obtain a transformed sugar beet cell; and
(c) regenerating a transgenic sugar beet plant from the transformed sugar beet plant cell.

Thus, a sugar beet plant is obtained which exhibits a durable resistance and in which the virus does not replicate. This is a unique aspect of the transformant of the invention and has not been disclosed before.

According to the invention the essential sequence homology of the fragment is a homology of at least 70%, preferably at least 80%, more preferably at least 90%, most preferably at least 95%.

The homology or "degree of similarity" is used to denote nucleotide sequences which when aligned have similar (identical or conservatively replaced) nucleotides in like positions or regions. For example, two nucleotide sequences with at least 85% homology to each other have at least 85% homologous (identical or conservatively replaced nucleotides) in a like position when aligned optimally allowing for up to 3 gaps, with the provison that in respect of the gaps a total of not more than 15 amino acid resides is affected. The degree of similarity may be determined using methods well known in the art (see, for example, Wilbur, W.J. and Lipman, D.J. "Rapid Similarity Searches of Nucleic Acid and Protein Data Banks." Proceedings of the National Academy of Sciences USA 80, 726-730 (1983) and Myers E.and Miller W. "Optimal Alignments in Linear Space". Comput. Appl. Biosci. 4:11-17(1988)). One programme which may be used in determining the degree of similarity is the MegAlign Lipman-Pearson one pair method (using default parameters) which can be obtained from DNAstar Inc, 1228, Selfpark Street, Madison, Wisconsin, 53715, USA as part of the Lasergene system. The test for homology of the sequence is based on the percent identity which is calculated by Fast DB based on the following parameters: mismatch penalty 1.0, gap penalty (1.00), gap size penalty 0.33 and joining penalty 30.0.

Preferred embodiments of the invention use various fragments having nucleic acid sequences that correspond with the homology indicated or completely to nucleotides 153 to 3258, 169 to 539, 1226 to 1683, 2754 to 3192 or to all of the 6746 nucleotides of RNA 1.

The present invention also includes DNA which hybridises to the DNA of the present invention and which codes for RNA1. Preferably, such hybridisation occurs at, or between, low and high stringency conditions. In general terms, low stringency conditions can be defined as 3 x SCC at about ambient temperature to about 65°C, and high stringency conditions as 0.1 x SSC at about 65°C. SSC is the name of a buffer of 0.15M NaCl, 0.015M trisodium citrate. 3 x SSC is three time as strong as SSC and so on.

The fragment can be introduced into a regenerable plant cell by means of a DNA vector harboring the fragment and transcription and translation regulatory sequences operably linked therewith using standard plant transformation methods such as Agrobacterium-mediated transformation of cells embedded in plant tissues such as cotyledons (Krens et al., Plant Science 116: 97-106; 1996) or polyethylene glycol-mediated DNA uptake of single cells like the guard cell protoplasts (Hall et al., Nature Biotechnology 14: 1133-1138; 1996). The DNA vector harboring the fragment is also part of the present invention.

The use of constructs constructed such that the gene sequence inhibits or promotes gene expression is quite well understood. A complete gene sequence, under the control of a promoter that operates effectively in the plant, will generally overexpress the gene product, leading to an amplification of the effect of the protein so produced. Sometimes the gene product is reduced: this phenomenon is termed "co-suppression". Downregulating this gene can be done by several techniques. It can be done by 'dominant-negative' constructs. These contain the specific DNA binding domain as well as possible dimerisation domains but are transcriptionally inactive. They 'sit' on the promoters of the target genes and thereby prevent the binding of the endogenous protein. Additionally, reduction of the gene product can also be obtained by using such dominant negative mutation, or by reversing the orientation of the gene sequence with respect to the promoter so that it produces a type of gene product called "antisense" messenger RNA.

A DNA construct according to the invention may be an "antisense" construct generating "antisense" RNA or a "sense" construct (encoding at least part of the functional protein) generating "sense" RNA.

"Antisense RNA" is an RNA sequence which is complementary to a sequence of bases in the corresponding mRNA: complementary in the sense that each base (or the majority of bases) in the antisense sequence (read in the 3' to 5' sense) is capable of pairing with the corresponding base (G with C, A with U) in the mRNA sequence read in the 5' to 3' sense. Such antisense RNA may be produced in the cell by transformation with an appropriate DNA construct arranged to generate a transcript with at least part of its sequence complementary to at least part of the coding strand of the relevant gene (or of a DNA sequence showing substantial homology therewith).

"Sense RNA" is an RNA sequence, which is substantially homologous to at least part of the corresponding mRNA sequence. Such sense RNA may be produced in the cell by transformation with an appropriate DNA construct arranged in the normal orientation so as to generate a transcript with a sequence identical to at least part of the coding strand of the relevant gene (or of a DNA sequence showing substantial homology therewith). Suitable sense constructs may be used to inhibit gene expression (as described in International Patent Publication WO91/08299).

DNA constructs according to the invention may comprise a base sequence at least 10 bases (preferably at least 35 bases) in length for transcription into RNA. There is no theoretical upper limit to the base sequence - it may be as long as the relevant mRNA produced by the cell - but for convenience it will generally be found suitable to use sequences between 100 and 1000 bases in length. The preparation of such constructs is described in more detail below.

As a source of the DNA base sequence for transcription, a suitable cDNA or genomic DNA, RNA or synthetic polynucleotide may be used. The transcriptional initiation region (or promoter) operative in plants may be a constitutive promoter (such as the 35S cauliflower mosaic virus promoter) or an inducible or developmentally regulated promoter, as circumstances require. Suitable DNA sequences for control of expression of the plant expressible genes (including marker genes), such as transcriptional initiation regions, enhancers, leader sequences, non-transcribed leaders and the like, may be derived from any gene that is expressible in a plant cell. Also hybrid promoters combining functional portions of various promoters, or synthetic equivalents thereof can be employed. Apart from constitutive promoters, inducible promoters, or promoters otherwise regulated in their expression pattern, e.g. developmentally or cell-type specific, may be used to control expression of the expressible genes according to the invention. For example, it may be desirable to modify protein activity at certain stages of the plant's development. Use of a constitutive promoter will tend to affect protein levels and functions in all parts of the plant, while use of a tissue-specific promoter allows more selective control of gene expression and affected functions.

Another option under this invention is to use inducible promoters. Promoters are known which are inducible by pathogens, by stress, by chemicals and by environmental signals. The induction of the gene activity by internal or external induction is within the scope of the present invention. Promoters of this type enable the inducibility of the gene activity in a controlled manner, thus the plant can develop normally without any undue influence by the transgene gene. Promoters that are inducible promoters include those described in DE 4446342 (fungus and auxin inducible PRP-1), WO 96/28561 (fungus inducible PRP-1), EP 0 712 273 (nematode inducible), EP 0 330 479 and US 5,510,474 (stress inducible), WO/96/12814 (cold inducible), and Zeneca's alcohol inducible promoter. Other inducible promoters are described in EP 0 494 724, EP 0 619 844, WO 92/19724. Thus the gene product, whether antisense or sense RNA or the peptide, is only produced in the tissue at the time when its action is required.

As mentioned above, the term "inducible promoter" includes promoters which may be induced chemically. The use of a promoter sequence which is controlled by the application of an external chemical stimulus is most especially preferred. The external chemical stimulus is preferably an agriculturally acceptable chemical, the use of which is compatible with agricultural practice and is not detrimental to plants or mammals. The inducible promoter region most preferably comprises an inducible switch promoter system such as, for example, a two component system such as the alcA/alcR gene switch promoter system described in the published International Publication No. WO 93/21334, the ecdysone switch system as described in the International Publication No. WO 96/37609 or the GST promoter as described in published International Patent Application Nos. WO 90/08826 and WO 93/031294, the teachings of which are incorporated herein by reference. Such promoter systems are herein referred to as "switch promoters". The switch chemicals used in conjunction with the switch promoters are agriculturally acceptable chemicals making this system particularly useful in the method of the present invention.

The skilled person will be capable of selecting a DNA vector for use in these methods. An example of a suitable vector for the Agrobacterium-mediated transformation is pBIN19. Suitable vectors for the PEG-mediated transformation include the pBluescript vector or pIGPD7 (Hall et al., Nature Biotechnology 14: 1133-1138; 1996). Introduction of the fragment into these vectors can be achieved by means of standard molecular biological techniques as for example described in Sambrook et al., "Molecular Cloning, A Laboratory Manual", Cold Spring Harbor Laboratory Press; 1989.

Transformed plants obtained by the method of the present invention show absolute resistance, or immunity, to BNYVV. In contrast, previous attempts to convey resistance to plants to BNYVV (Kallerhof et al., Plant Cell Reports 9: 224-228, 1990; and Mannerlof et al., Euphytica 90: 293-299, 1996) or other viruses, such as to tobacco mosaic virus (TMV) (Donson et al., Mol. Plant-Microbe Interact. 6: 635-642; 1993) by transforming plants with portions of the viral genome were less successful. Inoculated leafs still showed symptoms of infection, thus indicating that the resistance is not absolute. It is therefore surprising that the method of the invention is capable of conveying absolute resistance to BNYVV to sugarbeet plants.

Furthermore the invention relates to a transformed plant cell and a transgenic plant resistant to BNYVV as well as reproducible structures, such as seeds, calluses, buds, embryos, obtained from the transgenic plants and the progeny derived therefrom.

In a preferred embodiment of the invention the resistance described herein can be combined with other types of resistance or tolerance to BNYVV.

The invention will further be illustrated in the following examples and figures, but is not limited thereto.

Figure 1 shows a diagrammatic representation of the genomic organisation of Beet Necrotic Yellow Vein Virus (based on Jupin et al., Seminars in Virology vol 2.2: 112-129; 1991).

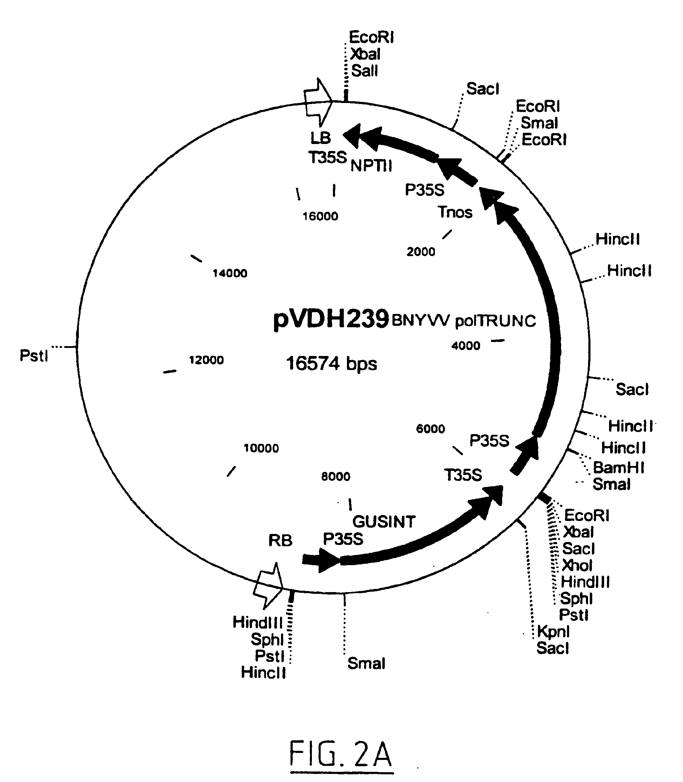

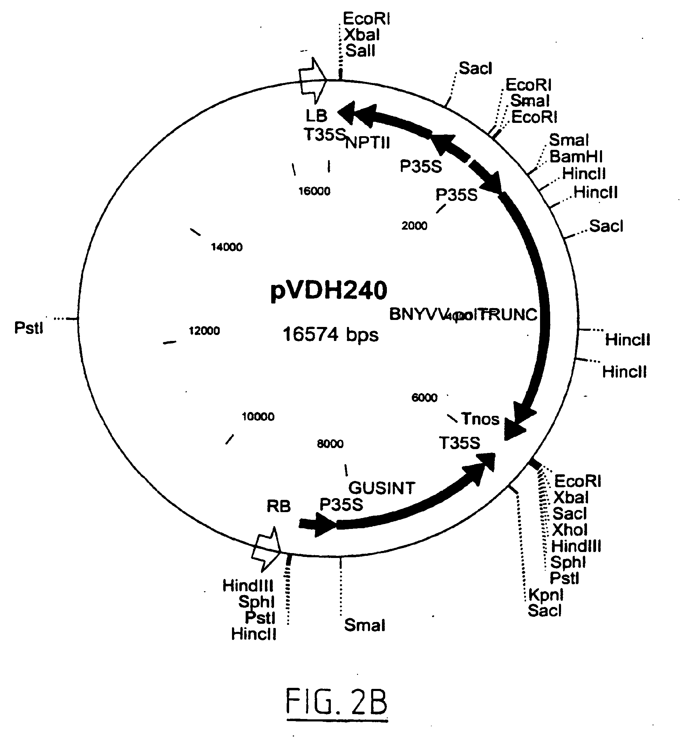

Figure 2 shows the physical maps of pVDH239 and pVDH240. LB=left border, RB=right border, P35S=CaMV 35S promoter, NPTII= neomycin phosphotransferase II, T35S=CaMV 35S polyadenylation signal, GUSINT=beta-glucuronidase gene, BNYVVpolTRUNC=BNYVV cDNA1 fragment, Tnos=nopaline synthase gene derived polyadenylation signal. The positions of the main restriction enzyme recognition sites are indicated.

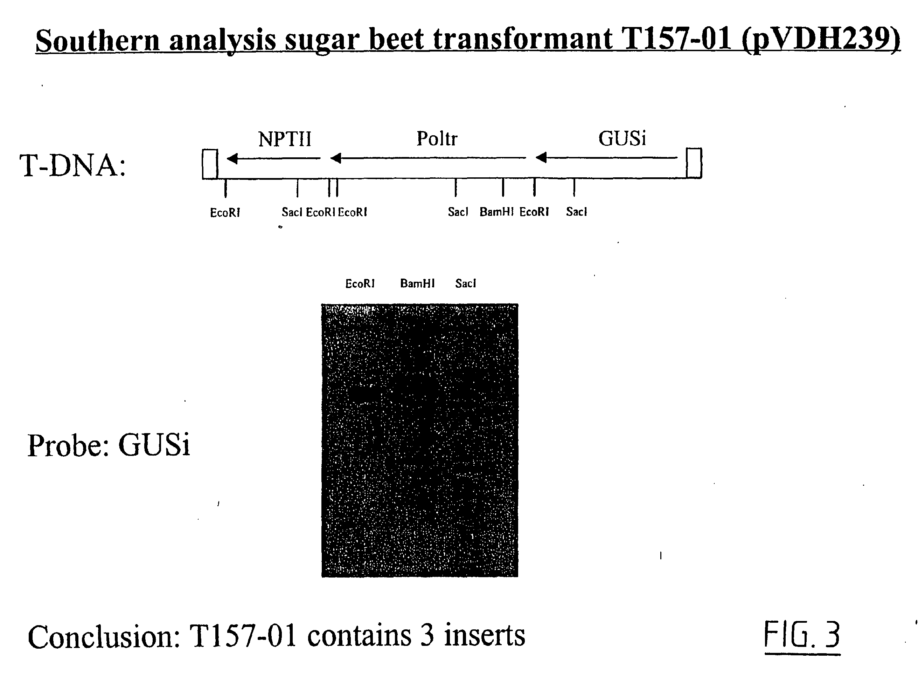

Figure 3 shows a Southern blot analysis by which the number of T-DNA insertions integrated into the genome of the primary sugar beet transformant T157-01 has been determined. The outline of the T-DNA structure of the binary vector pVDH239 is shown at the top.

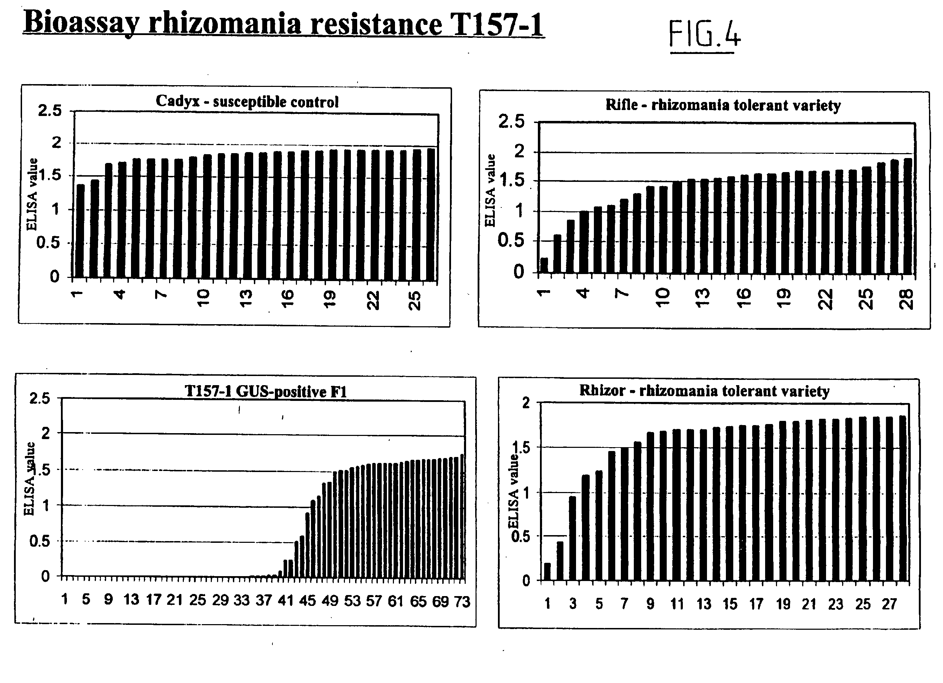

Figure 4 shows diagrams of the individual ELISA values of the root extracts of sugar beet plants of the populations Cadyx (susceptibel control), Rifle (rhizomania tolerant variety), Rhizor (rhizomania tolerant variety) and T157-01 (GUS-positive F1 individuals) after inoculation with BNYVV-infested soil. Each number at the horizontal axis represents an individual plant.

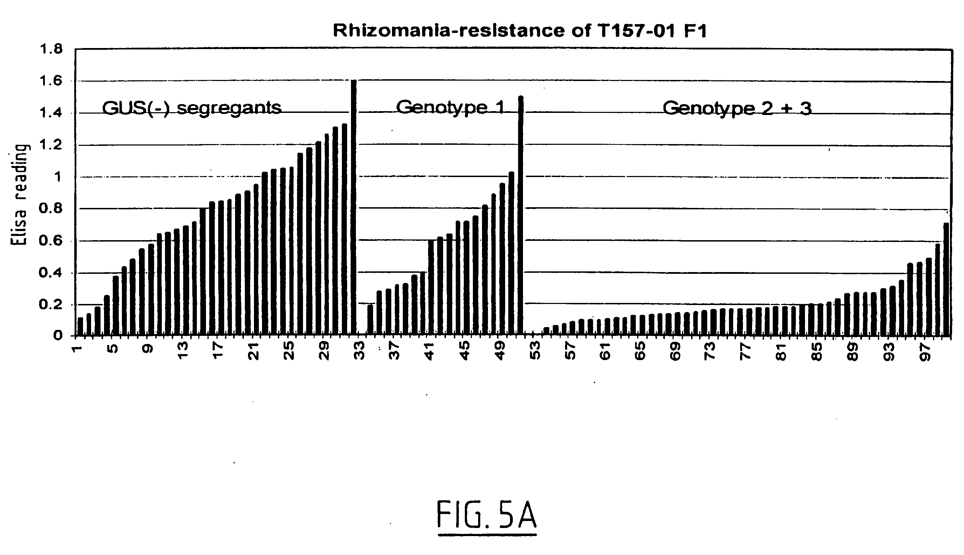

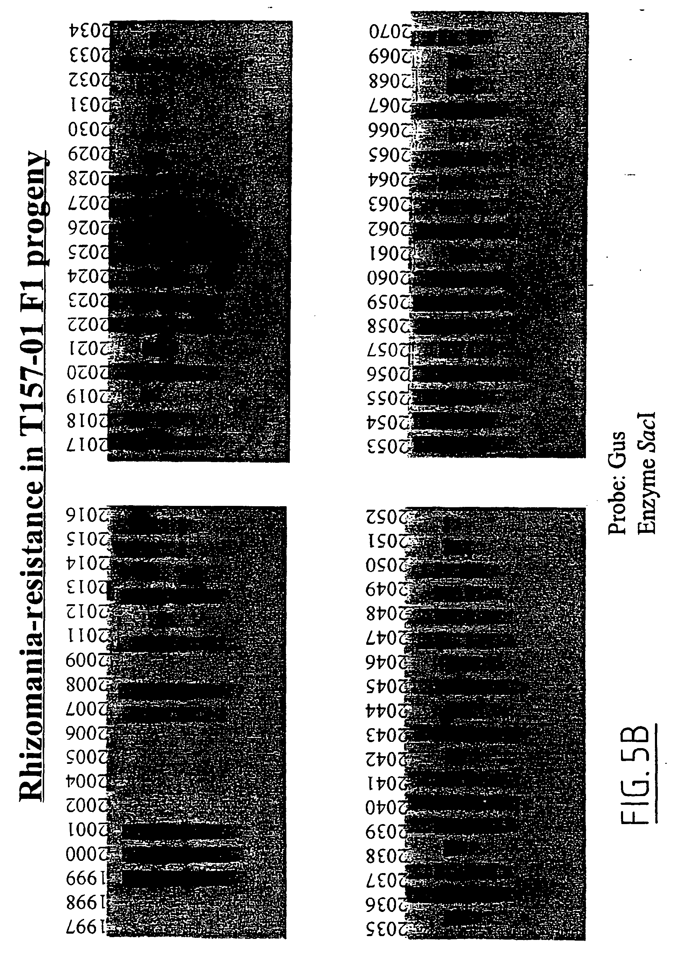

Figure 5B shows a Southern blot analysis by which the number of T-DNA insertions integrated into the genome of the F1 progeny plants of T157-01 has been determined as well as a diagram of the individual ELISA values of the root extracts of the F1 progeny plants of T157-01) after inoculation with BNYVV-infested soil (figure 5A). The numbers on top of the Southern blots represent the lab-codes of the individual F1 progeny plants. The ELISA values indicated by "Genotype 1" in the lower pannel correspond to the indivduals showing a single band in the Southern blot (2012, 2019, 2021, 2029, 2030, 2031, 2034, 2035, 2038, 2042, 2044, 2046, 2051, 2052, 2061, 2066, 2068, 2069), whereas the ELISA values indicated by "Genotype 2 + 3" in the lower pannel correspond to the individuals showing 2 or 3 bands in the Southern blot (1999, 2000, 2001, 2007, 2008, 2011, 2013, 2014, 2015, 2016, 2017, 2018, 2020, 2022, 2023, 2024, 2025, 2026, 2027, 2028, 2032, 2033, 2036, 2037, 2039, 2040, 2041, 2043, 2045, 2047, 2048, 2049, 2050, 2053, 2054, 2055, 2056, 2057, 2058, 2059, 2060, 2062, 2063, 2064, 2065, 2067, 2070). The ELISA values indicated by "GUS(-) segregants" in the lower pannel correspond to the individual F1 progeny plants which are GUS-negative (1997, 1998, 2002, 2004, 2005, 2006, 2009, rest not shown).

### EXAMPLES

### EXAMPLE 1

### Preparation of contruct with truncated BNYVV replicase sequence

Two primer combinations were used to obtain the cDNA clones of BNYVV for cloning in the tranformation vector (Bouzoubaa et al. J. Gen. Virol. 68:615-626; 1987).
For the 5'-end the primers were
P1: 5'-CGCGGATCCACCATGGCAGATTCGTTC-3' (containing a BamHI and NcoI restriction site and nucleotides identical to nucleotides 153-168), and
P2: 5'-GACGAATTCAAGTCGTCTTTC-3' (EcoRI restriction site and nucleotides complementary to nucleotides 288-301).
For the 3'-end the primers were
P3: 5'-GACGAATTCGAAAGATGAGTCTA-3' (EcoRI site and nucleotides identical to nucleotides 2799-2812), and
P4: 5'-CGCAGATCTTTAACTGCTCATCACCAAC-3' (BglII site and nucleotides complementary to nucleotides 3244-3258 and stop codon).

Before cloning the fragments into the Bluescript vector (Stratagene, La Jolla, CA, USA) the SalI/HincII/AccI site was replaced by a BglII site. After amplification of BNYVV cDNA using P1 and P2 a DNA fragment was obtained which was digested with BamHI and EcoRI and inserted into the modified, BamHI/EcoRI cleaved pBleuscript vector to yield pKSNBPol1. Subsequently, BNYVV cDNAl was amplified using P3 and P4. The resulting DNA fragment was cleaved by EcoRI and BglII and inserted into pKSBNPol1 cleaved with EcoRI and BglII, yielding pKSNBPpol2.

Thereafter the AccI fragment of BNYVV cDNA1 identical to nucleotides 250-2815 was cloned in AccI cleaved pKSBNPol2. Thus, a pKSBNPoltrunc was obtained which comprised the BNYVV cDNA1 fragment (poltrunc) identical to the nucleotides 153-3258, flanked by a BamHI site at the 5'-end and by a BglII fragment at the 3'-end. The poltrunc fragment was cloned as BamHI-Bglll fragment in the BamHI site of pVDH4 such that a functional sense poltrunc fragment was placed behind the CaMV 35S promoter and before the nopaline terminator sequence. The complete construct, carrying the CaMV35S promoter the poltrunc fragment and nopaline terminator sequence was excised from the plasmid with ClaI and cloned in the binary transformation vector pVDH212 in which the BamHI site was converted into a ClaI site by inserting a molecular linker.

pVDH212 is a pBIN19 derived binary transformation vector, which contains 35S-NPTII (neomycin phosphotransferase under control of the CaMV 35S promoter as a selectable marker gene conferring resistance to kanamycin), as well as 35S-GUSi (gene encoding beta-glucuronidase (Vancanneyt et al., Mol. Gen. Genet. 220: 245-250; 1990). Thus, two different transformation vectors containing the poltrunc construct were obtained: one, pVDH239, with the poltrunc construct in the same transcriptional direction as the NPTII and GUS gene, and another, pVDH240, with the poltrunc construct having_an opposite transcriptional direction. The binary transformation vectors pVDH239 and 240 (figure 2) were conjugated and transferred to Agrobacterium tumefaciens LBA4404 (Phabagen Collection, Utrecht, the Netherlands) resulting in HAT1239 (for pVDH239) and HAT1240 (for pVDH240) strains. HAT1239 and HAT1240 were used for transformation of sugar beet.

### EXAMPLE 2

### Transformation of sugar beet

Methods for Agrobacterium-mediated transformation using binary vectors are well established and known to a person skilled in the art.

To obtain transformed sugar beet plants, O-type B8M5.9 plants were transformed according to Krens et al. (Plant Science 116: 97-106; 1996). Transformation with Agrobacterium HAT1239 yielded 8 transformants: T156-03, T156-06, T156-09, T156-10, T156-13, T156-20, T150-30, and T157-01, and with Agrobacterium HAT1240 12 transformants were obtained: T163-01, T183-02, T183-06, T183-10, T183-16, T183-19, T183-21, T183-23, T183-26, T184-02, T184-03 and T184-04. The transformants were induced to flowering by vernalisation and used to produce seeds either through self-pollination (selfing or S1 seed) or cross-pollination on male sterile plants (hybrid or F1 seed). The pollination was performed in closed pollen chambers to prevent pollination by sugar beet pollen from a different source.

The F1 seeds were used as starting material to carry out a bioassay for BNYW resistance. The seedlings were screened initially for GUS activity which indicates the presence of the T-DNA. GUS-negative segregants were used as a negative control in the bioassay. Depending on the total number of T-DNA inserts, the resistance, if present, can segregate within the GUS-positive population. Only T157-01 derived progeny showed BNYVV resistance.

Subsequently, the primary transformant T157-01 was analysed in further detail on Southern blot. Genomic DNA was isolated from leaves, digested seperately with EcoR1, BamHI and SacI and used to prepare a Southern blot which was subsequently probed with GUS. From this experiment it was concluded that the primary transformant contained three T-DNA inserts (figure 3).

### EXAMPLE 3

### Bioassay

A lateral root bioassay was developed as a single plant test for the selection of rhizomania resistant sugarbeet plants. One-week-old plantlets were transplanted in a standard mixture of 10% rhizomania infected soil and further incubated for 4 weeks. The conditions for infection were optimised and standardised on the level of the infection pressure and the growth of rootlets (only formation of hairy roots). Incubation conditions were: 18.9-19°C day/night, 70% relative humidity, 10.000 lux white light, pots at 32.5 cm from the lamps.

The amount of virus in the rootlets which was directly correlated with the resistance mechanism in the plant was measured by an ELISA method. The cut-off value for resistant plants was put at an OD-value of 0.2 (Clark et al. J. Virol. Meth. 15:213-222; 1987). Four weeks after transplantation the lower part of the roots was used for ELISA. The root piece was dried on a filter paper and transferred to a mortar. Extraction buffer was added at a ratio of 10 volume equivalents of the root weight (dilution 1/10). Root juice was extracted by hand crushing. The juice was transferred to 1.5 ml tubes and centrifuged (300 rpm, 10 minutes) the supernatants were kep on ice and assayed. Populations tested were:

| Population | Number of plants | Remarks |
|---|---|---|
| T 157-01 (F1) | 73 | GUS(+) individuals |
| Cadyx F 052 | 26 | sensitive control |
| Rhizor F 202 | 27 | tolerant control |
| Rifle | 28 | tolerant control |

Within the group of T157-01 GUS(+) plants two categories can be observed (figure 4). One category displays immunity to BNYVV (ELISA cut-off 0.2) with an average ELISA value of 0.006 which is the background level of the experimental system. The other category shows normal susceptibility with an average ELISA value in the range of the susceptible control.

The infection pressure in this bioassay was high due to the increase of temperature during a part of the infection period. Consequently there was no clear distinction between Cadyx and Rizor/Rifle (figure 4). On the other hand, the resistant plants selected in the T 157-01 F1 progeny could be considered as totally resistant independent of the rhizomania infection pressure (figure 4). In conclusion, the introduced construct containing the BNYVV cDNA1 fragment resulted in a dramatic negative effect on the multiplication of BNYVV in the lateral roots of the inoculated transgenic sugar beet plants which effectively rendered the plants completely resistant to rhizomania.

In order to explain the segregation of the GUS(+) population in a resistant and a suceptible category the individual plants were analysed in a separate experiment for resistance to BNYVV and simultaneously for the presence of the T-DNAs by Southern analysis using SacI as restriction enzyme and GUS as a probe. The results, as depicted in Figure 5A and 5B, point out that the GUS(+)_plants within this population which contained a single band on the Southern blot were all susceptible (average ELISA value 0.63), whereas the GUS(+) plants which contained either 2 or 3 bands on the Southern blot were all resistant (average ELISA value: 0.21). The GUS(-) segregants were all susceptible (average ELISA value 0.80). Apparently the resistant phenotype is linked to the presence of the upper and lower band of this particular band pattern obtained on Southern blot, whereas the presence of the middle band is not linked to the resistant phenotype. It is therefore concluded that the segregation of the GUS(+) population into a suseptible and a resistant category can be explained by the fact that one of the T-DNAs which can result in a GUS(+) phenotype does not contribute to the resistance to BNYVV.

## Claims

1. Method for conveying resistance to beet necrotic yellow vein virus (BNYVV) to a sugar beet plant, comprising the following steps:
(a) preparing a DNA fragment of at least 15 nucleotides in a sequence that is at least 70% homologous to the corresponding nucleotide sequence of the genomic RNA 1 of the beet necrotic yellow vein virus (BNYVV),
(b) introducing said DNA fragment. Operably linked to a promoter that is active in sugar beet plants, into a sugar beet plant cell to obtain a transformed sugar beet cell; and
(c) regenerating a transgenic sugar beet plant from the transformed sugar beet plant cell.

2. Method according to claim 1, wherein the DNA fragment is at least 80%, preferably at least 90%, more preferably at least 95% homologous to the corresponding nucleotide sequence of the genomic RNA 1 of said virus.

3. Method according to claim 1 or 2, **characterized in that** the DNA fragment is at least 35 bases in length.

4. Method according to any of claims 1 to 3, **characterized in that** the fragment has a nucleic acid sequence that corresponds with the homology indicated in claims 1 and 2 to nucleotides 153 to 3258 of RNA 1 of said virus.

5. Method according to any of claims 1 to 3, **characterized in that** the fragment is between 100 and 1000 bases in length.

6. Method according to claim 5, **characterized in that** the fragment has a nucleic acid sequence that corresponds with the homology indicated in claims 1 and 2 to nucleotides 169 to 539 of RNA 1 of said virus.

7. Method according to claim 5, **characterized in that** the fragment has a nucleic acid sequence that corresponds with the homology indicated in claims 1 and 2 to nucleotides 1226 to 1683 of RNA 1 of said virus.

8. Method according to claim 5, **characterized in that** the fragment has a nucleic acid sequence that corresponds with the homology indicated in claims 1 and 2 to nucleotides 2754 to 3192 of RNA 1 of said virus.

9. Method according to any of claims 1 to 3, **characterized in that** the fragment consists of 6746 nucleotides.

10. Method as claimed in claims 1-9 **characterized in that** the fragment is introduced into the cell by means of a DNA vector harboring the fragment and transcription and translation regulatory sequences operably linked therewith.

11. Transformation vector for conveying resistance to BNYVV to a plant, harboring a fragment of at least 15 nucleotides in a sequence that is at least 70% homologous to the corresponding nucleotide sequence of the genomic RNA 1 of the beet necrotic yellow vein virus (BNYVV), which is operably linked to a promoter that is active in sugar beet plants.

12. Vector according to claim 11, wherein the fragment is at least 80%, preferably at least 90%, more preferably at least 95% homologous to the corresponding nucleotide sequence of the genomic RNA 1 of said virus.

13. Vector according to claim 11 or 12, wherein the fragment is at least 35 bases in length.

14. Vector according to any of claims 11 to 13, **characterized in that** the fragment has a nucleic acid sequence that corresponds with the homology indicated in claims 11 and 12 to nucleotides 153 to 3258 of RNA 1 of said virus.

15. Vector according to any of claims 11 to 13, wherein the fragment is between 100 and 1000 bases in length.

16. Vector according to claim 15, **characterized in that** the fragment has a nucleic acid sequence that corresponds with the homology indicated in claims 11 and 12 to nucleotides 169 to 539 of RNA 1 of said virus.

17. Vector according to claim 15, **characterized in that** the fragment has a nucleic acid sequence that corresponds with the homology indicated in claims 11 and 12 to nucleotides 1226 to 1683 of RNA 1 of said virus.

18. Vector according to claim 15, **characterized in that** the fragment has a nucleic acid sequence that corresponds with the homology indicated in claims 11 and 12 to nucleotides 2754 to 3192 of RNA 1 of said virus.

19. Vector according to any of claims 11 to 13, **characterized in that** the fragment consists of 6746 nucleotides.

20. Use of a vector as claimed in claims 11 to 19 for the transformation of a plant cell.

21. Plant cell, exhibiting a resistance to BNYVV, comprising in its genome a DNA fragment of at least 15 nucleotides in a sequence which is at least 70% homologous to the corresponding nucleotide sequence of the genomic RNA 1 of said virus.

22. Plant cell as claimed in claim 21, wherein the fragment is at least 80%, preferably at least 90%, more preferably at least 95% homologous to the corresponding nucleotide sequence of the genomic RNA 1 of said virus.

23. Plant cell as claimed in claim 21 or 22, wherein the fragment is at least 35 bases in length.

24. Plant cell according to any of claims 21 to 23, **characterized in that** the fragment has a nucleic acid sequence that corresponds with the homology indicated in claims 21 and 22 to nucleotides 153 to 3258 of RNA 1 of said virus.

25. Plant cell as claimed in any of claims 21 to 23, wherein the fragment is between 100 and 1000 bases in length.

26. Plant cell according to claim 25, **characterized in that** the fragment has a nucleic acid sequence that corresponds with the homology indicated in claims 21 and 22 to nucleotides 169 to 539 of RNA 1 of said virus.

27. Plant cell according to claim 25, **characterized in that** the fragment has a nucleic acid sequence that corresponds with the homology indicated in claims 21 and 22 to nucleotides 1226 to 1683 of RNA 1 of said virus.

28. Plant cell according to claim 25, **characterized in that** the fragment has a nucleic acid sequence that corresponds with the homology indicated in claims 21 and 22 to nucleotides 2754 to 3192 of RNA 1 of said virus.

29. Plant cell according to any of claims 21 to 23, **characterized in that** the fragment consists of 6746 nucleotides.

30. Plant cell as claimed in claims 21-29 being part of a sugar beet plant that is resistant against BNYVV.

31. Use of a plant cell as claimed in claims 21 to 29 for the regeneration therefrom of a sugar beet plant that is resistant against BNYVV.

32. Sugar beet plant, exhibiting a resistance to BNYVV, consisting at least partly of plant cells as claimed in claims 21-29.

33. BNYVV resistant progeny of a sugar beet plant as claimed in claim 32, **characterized in that said** progeny consists at least partly of plant cells according to claims 21-29.

34. Seeds of a sugar beet plant as claimed in claim 32 or of progeny according to claim 33, **characterized in that** said seeds consist at least partly of cells according to claims 21-29, and can be regenerated into a plant exhibiting a resistance to BNYVV.

35. Vegetatively reproducible structures, such as calluses, buds, embryos, obtained from a plant according to claim 32 or progeny according to claim 33, **characterized in that** said structures consist at least partly of cells according to claims 21-29, and can be regenerated into a plant exhibiting a resistance to BNYVV.

## Patentansprüche

1. Verfahren, um einer Zuckerrübenpflanze Resistenz gegen das Beet Necrotic Yellow Vein Virus (Aderngelbfleckigkeitsvirus der Rübe) zu verleihen, welches folgende Schritte umfasst:
(a) Herstellen eines DNA-Fragmentes aus mindestens 15 Nukleotiden in einer Sequenz, die zu mindestens 70% homolog zu der entsprechenden Nukleotidsequenz der genomischen RNA 1 des Beet Necrotic Yellow Vein Virus (BNYVV) ist,
(b) Einführen des DNA-Fragmentes, das funktionell mit einem Promotor verknüpft ist, der in Zuckerrübenpflanzen aktiv ist, in eine Zuckerrübenpflanzenzelle, um eine transformierte Zuckerrübenzelle zu erhalten; und
(c) Regenerieren einer transgenen Zuckerrübenpflanze aus der transformierten Zuckerrübenpflanzenzelle.

2. Verfahren nach Anspruch 1, wobei das DNA-Fragment zu mindestens 80%, vorzugsweise zu mindestens 90%, mehr bevorzugt zu mindestens 95% homolog zu der entsprechenden Nukleotidsequenz der genomischen RNA 1 des Virus ist.

3. Verfahren nach Anspruch 1 oder 2, **dadurch gekennzeichnet, dass** das DNA-Fragment eine Länge von mindestens 35 Basen aufweist.

4. Verfahren nach einem der Ansprüche 1 bis 3, **dadurch gekennzeichnet, dass** das Fragment eine Nukleinsäuresequenz aufweist, die der in Anspruch 1 und 2 angegebenen Homologie zu Nukleotid 153 bis 3258 der RNA 1 des Virus entspricht.

5. Verfahren nach einem der Ansprüche 1 bis 3, **dadurch gekennzeichnet, dass** das Fragment eine Länge zwischen 100 und 1000 Basen hat.

6. Verfahren nach Anspruch 5, **dadurch gekennzeichnet, dass** das Fragment eine Nukleinsäuresequenz aufweist, die der in Anspruch 1 und 2 angegebenen Homologie zu Nukleotid 169 bis 539 der RNA 1 des Virus entspricht.

7. Verfahren nach Anspruch 5, **dadurch gekennzeichnet, dass** das Fragment eine Nukleinsäuresequenz aufweist, die der in Anspruch 1 und 2 angegebenen Homologie zu Nukleotid 1226 bis 1683 der RNA 1 des Virus entspricht.

8. Verfahren nach Anspruch 5, **dadurch gekennzeichnet, dass** das Fragment eine Nukleinsäuresequenz aufweist, die der in Anspruch 1 und 2 angegebenen Homologie zu Nukleotid 2754 bis 3192 der RNA 1 des Virus entspricht.

9. Verfahren nach einem der Ansprüche 1 bis 3, **dadurch gekennzeichnet**, das das Fragment aus 6746 Nukleotiden besteht.

10. Verfahren nach Anspruch 1-9, **dadurch gekennzeichnet, dass** das Fragment in die Zelle mittels eines DNA-Vektors eingeführt wird, welcher das Fragment und transkriptions- und translationsregulierende Sequenzen aufweist, die damit funktionell verknüpft sind.

11. Transformationsvektor, um einer Pflanze Resistenz gegen BNYVV zu verleihen, welcher ein Fragment von mindestens 15 Nukleotiden in einer Sequenz aufweist, die zu der entsprechenden Nukleotidsequenz der genomischen RNA 1 des Beet Necrotic Yellow Vein Virus (BNYVV) zu mindestens 70% homolog ist, welches funktionell mit einem Promotor verknüpft ist, der in Zuckerrübenpflanzen aktiv ist.

12. Vektor nach Anspruch 11, wobei das Fragment zu mindestens 80%, vorzugsweise zu mindestens 90%, mehr bevorzugt zu mindestens 95% homolog zu der entsprechenden Nukleotidsequenz der genomischen RNA 1 des Virus ist.

13. Vektor nach Anspruch 11 oder 12, wobei das Fragment eine Länge von mindestens 35 Basen aufweist.

14. Vektor nach einem der Ansprüche 11 bis 13, **dadurch gekennzeichnet, dass** das Fragment eine Nukleinsäuresequenz aufweist, die der in Anspruch 11 und 12 angegebenen Homologie zu Nukleotid 153 bis 3528 der RNA 1 des Virus entspricht.

15. Vektor nach einem der Ansprüche 11 bis 13, wobei das Fragment eine Länge zwischen 100 und 1000 Basen aufweist.

16. Vektor nach Anspruch 15, **dadurch gekennzeichnet, dass** das Fragment eine Nukleinsäuresequenz aufweist, die der in Anspruch 11 und 12 angegebenen Homologie zu Nukleotid 169 bis 539 der RNA 1 des Virus entspricht.

17. Vektor nach Anspruch 15, **dadurch gekennzeichnet, dass** das Fragment eine Nukleinsäuresequenz aufweist, die der in Anspruch 11 und 12 angegebenen Homologie zu Nukleotid 1226 bis 1683 der RNA 1 des Virus entspricht.

18. Vektor nach Anspruch 15, **dadurch gekennzeichnet, dass** das Fragment eine Nukleinsäuresequenz aufweist, die der in Anspruch 11 und 12 angegebenen Homologie zu Nukleotid 2754 bis 3192 der RNA 1 des Virus entspricht.

19. Vektor nach einem der Ansprüche 11 bis 13, **dadurch gekennzeichnet, dass** das Fragment aus 6746 Nukleotiden besteht.

20. Verwendung eines Vektors wie in Anspruch 11 bis 19 beansprucht für die Transformation einer Pflanzenzelle.

21. Pflanzenzelle, die eine Resistenz gegen BNVYY aufweist, welche in ihrem Genom ein DNA-Fragment von mindestens 15 Nukleotiden in einer Sequenz aufweist, die zu mindestens 70% zu der entsprechenden Nukleotidsequenz der genomischen RNA 1 des Virus homolog ist.

22. Pflanzenzelle wie in Anspruch 21 beansprucht, wobei das Fragment zu mindestens 80%, vorzugsweise zu mindestens 90%, mehr bevorzugt zu mindestens 95% homolog zu der entsprechenden Nukleotidsequenz der genomischen RNA 1 des Virus ist.

23. Pflanzenzelle wie in Anspruch 21 oder 22 beansprucht, wobei das Fragment eine Länge von mindestens 35 Basen aufweist.

24. Pflanzenzelle nach einem der Ansprüche 21 bis 23, **dadurch gekennzeichnet, dass** das Fragment eine Nukleinsäuresequenz aufweist, die der in Anspruch 21 und 22 angegebenen Homologie zu Nukleotid 153 bis 3258 der RNA 1 des Virus entspricht.

25. Pflanzenzelle wie in einem der Ansprüche 21 bis 23 beansprucht, wobei das Fragment eine Länge zwischen 100 und 1000 Basen aufweist.

26. Pflanzenzelle nach Anspruch 25, **dadurch gekennzeichnet, dass** das Fragment eine Nukleinsäuresequenz aufweist, die der in Anspruch 21 und 22 angegebenen Homologie zu Nukleotid 169 bis 539 der RNA 1 des Virus entspricht.

27. Pflanzenzelle nach Anspruch 25, **dadurch gekennzeichnet, dass** das Fragment eine Nukleinsäuresequenz aufweist, die der in Anspruch 21 und 22 angegebenen Homologie zu Nukleotid 1226 bis 1683 der RNA 1 des Virus entspricht.

28. Pflanzenzelle nach Anspruch 25, **dadurch gekennzeichnet, dass** das Fragment eine Nukleinsäuresequenz aufweist, die der in Anspruch 21 und 22 angegebenen Homologie zu Nukleotid 2754 bis 3192 der RNA 1 des Virus entspricht.

29. Pflanzenzelle nach einem der Ansprüche 21 bis 23, **dadurch gekennzeichnet, dass** das Fragment aus 6746 Nukleotiden besteht.

30. Pflanzenzelle wie in Anspruch 21-29 beansprucht, die Teil einer gegen BNYVV resistenten Zuckerrübenpflanze ist.

31. Verwendung einer Pflanzenzelle, wie in Anspruch 21 bis 29 beansprucht, für ihre Regeneration aus einer gegen BNYVV resistenten Zuckerrübenpflanze.

32. Zuckerrübenpflanze, die eine Resistenz gegen BNYVV aufweist, die zumindest teilweise aus Pflanzenzellen wie in Anspruch 21-29 beansprucht besteht.

33. BNYVV-resistente Nachkommen einer Zuckerrübenpflanze wie in Anspruch 32 beansprucht, **dadurch gekennzeichnet, dass** die Nachkommen zumindest teilweise aus Pflanzenzellen nach Anspruch 21-29 bestehen.

34. Samen einer Zuckerrübenpflanze wie in Anspruch 32 beansprucht oder von Nachkommen nach Anspruch 33, **dadurch gekennzeichnet, dass** die Samen zumindest teilweise aus Zellen nach Anspruch 21-29 bestehen und zu einer Pflanze regeneriert werden können, die Resistenz gegen BNYVV aufweist.

35. Vegetativ reproduzierbare Strukturen, wie beispielsweise Kalli, Knospen, Embryonen, die aus einer Pflanze nach Anspruch 32 oder Nachkommen nach Anspruch 33 erhalten wurden, **dadurch gekennzeichnet, dass** die Strukturen zumindest teilweise aus Zellen nach Anspruch 21-29 bestehen, und zu einer Pflanze regeneriert werden können, die Resistenz gegen BNYVV aufweist.

## Revendications

1. Procédé pour conférer une résistance au virus des nervures jaunes et nécrotiques de la betterave (BNYVV) à une betterave sucrière, comprenant les étapes suivantes :
(a) préparer un fragment ADN d'au moins 15 nucléotides dans une séquence qui présente au moins 70 % d'homologie avec la séquence de nucléotides correspondante de l'ARN 1 génomique du virus des nervures jaunes et nécrotiques de la betterave (BNYVV),
(b) introduire ledit fragment ADN, en liaison opérationnelle avec un promoteur qui est actif dans des betteraves sucrières, dans une cellule de betterave sucrière afin d'obtenir une cellule de betterave sucrière transformée ; et
(c) régénérer une betterave sucrière transgénique à partir de la cellule de betterave sucrière transformée.

2. Procédé selon la revendication 1, dans lequel le fragment ADN présente au moins 80 %, de préférence au moins 90 %, de manière davantage préférée au moins 95 % d'homologie avec la séquence de nucléotides correspondante de l'ARN 1 génomique dudit virus.

3. Procédé selon la revendication 1 ou 2, **caractérisé en ce que** le fragment ADN a une longueur d'au moins 35 bases.

4. Procédé selon l'une quelconque des revendications 1 à 3, **caractérisé en ce que** le fragment possède une séquence d'acide nucléique qui correspond à l'homologie indiquée dans les revendications 1 ou 2 avec les nucléotides 153 à 3258 de l'ARN 1 dudit virus.

5. Procédé selon l'une quelconque des revendications 1 à 3, **caractérisé en ce que** le fragment a une longueur comprise entre 100 et 1000 bases.

6. Procédé selon la revendication 5, **caractérisé en ce que** le fragment possède une séquence d'acide nucléique qui correspond à l'homologie indiquée dans les revendications 1 et 2 avec les nucléotides 169 à 539 de l'ARN 1 dudit virus.

7. Procédé selon la revendication 5, **caractérisé en ce que** le fragment possède une séquence d'acide nucléique qui correspond à l'homologie indiquée dans les revendications 1 et 2 avec les nucléotides 1226 à 1683 de l'ARN 1 dudit virus.

8. Procédé selon la revendication 5, **caractérisé en ce que** le fragment possède une séquence d'acide nucléique qui correspond à l'homologie indiquée dans les revendications 1 et 2 avec les nucléotides 2754 à 3192 de l'ARN 1 dudit virus.

9. Procédé selon l'une quelconque des revendications 1 à 3, **caractérisé en ce que** le fragment consiste en 6746 nucléotides.

10. Procédé selon les revendications 1 à 9, **caractérisé en ce que** le fragment est introduit dans la cellule au moyen d'un vecteur ADN comportant le fragment et des séquences de régulation de la transcription et de la traduction en liaison opérationnelle avec celui-ci.

11. Vecteur de transformation pour conférer une résistance au BNYVV à une plante, comportant un fragment d'au moins 15 nucléotides dans une séquence qui présente au moins 70 % d'homologie avec l'ARN 1 génomique du virus des nervures jaunes et nécrotiques de la betterave (BNYVV), qui est en liaison opérationnelle avec un promoteur qui est actif dans les betteraves sucrières.

12. Vecteur selon la revendication 11, dans lequel le fragment présente au moins 80 %, de préférence au moins 90 %, de manière davantage préférée au moins 95 % d'homologie avec la séquence de nucléotides correspondante de l'ARN 1 génomique dudit virus.

13. Vecteur selon la revendication 11 ou 12, dans lequel le fragment a une longueur d'au moins 35 bases.

14. Vecteur selon l'une quelconque des revendications 11 à 13, **caractérisé en ce que** le fragment possède une séquence d'acide nucléique qui correspond à l'homologie indiquée dans les revendications 11 et 12 avec les nucléotides 153 à 3258 de l'ARN 1 dudit virus.

15. Vecteur selon l'une quelconque des revendications 11 à 13, dans lequel le fragment a une longueur comprise entre 100 et 1000 bases.

16. Vecteur selon la revendication 15, **caractérisé en ce que** le fragment possède une séquence d'acide nucléique qui correspond à l'homologie indiquée dans les revendications 11 et 12 avec les nucléotides 169 à 539 de l'ARN 1 dudit virus.

17. Vecteur selon la revendication 15, **caractérisé en ce que** le fragment possède une séquence d'acide nucléique qui correspond à l'homologie indiquée dans les revendications 11 et 12 avec les nucléotides 1226 à 1683 de l'ARN 1 dudit virus.

18. Vecteur selon la revendication 15, **caractérisé en ce que** le fragment possède une séquence d'acide nucléique qui correspond à l'homologie indiquée dans les revendications 11 et 12 avec les nucléotides 2754 à 3192 de l'ARN 1 dudit virus.

19. Vecteur selon l'une quelconque des revendications 11 à 13, **caractérisé en ce que** le fragment consiste en 6746 nucléotides.

20. Utilisation d'un vecteur selon les revendications 11 à 19 pour la transformation d'une cellule végétale.

21. Cellule végétale, exhibant une résistance au BNYVV, comprenant dans son génome un fragment ADN d'au moins 15 nucléotides dans une séquence qui présente au moins 70 % d'homologie avec la séquence de nucléotides correspondante de l'ARN 1 génomique dudit virus.

22. Cellule végétale selon la revendication 21, dans laquelle le fragment présente au moins 80 %, de préférence au moins 90 %, de manière davantage préférée au moins 95 % d'homologie avec la séquence de nucléotides correspondante de l'ARN 1 génomique dudit virus.

23. Cellule végétale selon la revendication 21 ou 22, dans laquelle le fragment a une longueur d'au moins 35 bases.

24. Cellule végétale selon l'une quelconque des revendications 21 à 23, **caractérisée en ce que** le fragment possède une séquence d'acide nucléique qui correspond à l'homologie indiquée dans les revendications 21 et 22 avec les nucléotides 153 à 3258 de l'ARN 1 dudit virus.

25. Cellule végétale selon l'une quelconque des revendications 21 à 23, dans laquelle le fragment a une longueur comprise entre 100 et 1000 bases.

26. Cellule végétale selon la revendication 25, **caractérisée en ce que** le fragment possède une séquence d'acide nucléique qui correspond à l'homologie indiquée dans les revendications 21 et 22 avec les nucléotides 169 à 539 de l'ARN 1 dudit virus.

27. Cellule végétale selon la revendication 25, **caractérisée en ce que** le fragment possède une séquence d'acide nucléique qui correspond à l'homologie indiquée dans les revendications 21 et 22 avec les nucléotides 1226 à 1683 de l'ARN 1 dudit virus.

28. Cellule végétale selon la revendication 25, **caractérisée en ce que** le fragment possède une séquence d'acide nucléique qui correspond à l'homologie indiquée dans les revendications 21 et 22 avec les nucléotides 2754 à 3192 de l'ARN 1 dudit virus.

29. Cellule végétale selon l'une quelconque des revendications 21 à 23, **caractérisée en ce que** le fragment consiste en 6746 nucléotides.

30. Cellule végétale selon les revendications 21 à 29 faisant partie d'une betterave sucrière qui est résistante contre le BNYVV.

31. Utilisation d'une cellule végétale selon les revendications 21 à 29 pour la régénération d'une betterave sucrière qui est résistante contre le BNYVV à partir de celle-ci.

32. Betterave sucrière, exhibant une résistance au BNYVV, consistant en partie du moins en des cellules végétales selon les revendications 21 à 29.

33. Progénie résistante au BNYVV d'une betterave sucrière selon la revendication 32, **caractérisée en ce que** ladite progénie consiste en partie du moins en des cellules végétales selon les revendications 21 à 29.

34. Graines d'une betterave sucrière selon la revendication 32 ou d'une progénie selon la revendication 33, **caractérisées en ce que** lesdites graines consistent en partie du moins en des cellules selon les revendications 21 à 29, et peuvent être régénérées en une plante exhibant une résistance au BNYVV.

35. Structures reproductibles de manière végétative, tel que des cals, des bourgeons, des embryons, obtenues à partir d'une plante selon la revendication 32 ou d'une progénie selon la revendication 33, **caractérisées en ce que** lesdites structures consistent en partie du moins en des cellules selon les revendications 21 à 29, et peuvent être régénérées en une plante exhibant une résistance au BNYVV.


### SEQUENCE LISTING

<110> SES Europe N.V./S.A.
<120> Method for conveying BNYVV resistance to sugar beet plants
<130> seseurope4seqlist
<140> PCT/EP00/00609
   <141> 2000-01-26
<150> EP 992002360
   <151> 1999-01-27
<160> 4
<170> PatentIn Ver. 2.1
<210> 1
   <211> 27
   <212> DNA
   <213> Artificial Sequence
<220>
   <223> Description of Artificial Sequence:primer P1
<400> 1
   cgcggatcca ccatggcaga ttcgttc 27
<210> 2
   <211> 21
   <212> DNA
   <213> Artificial Sequence
<220>
   <223> Description of Artificial Sequence: primer P2
<400> 2
   gacgaattca agtcgtcttt c 21
<210> 3
   <211> 23
   <212> DNA
   <213> Artificial Sequence
<220>
   <223> Description of Artificial Sequence: primer P3
<400> 3
   gacgaattcg aaagatgagt cta 23
<210> 4
   <211> 28
   <212> DNA
   <213> Artificial Sequence
<220>
   <223> Description of Artificial Sequence: primer P4
<400> 4
   cgcagatctt taactgctca tcaccaac 28